# EUROPEAN PATENT APPLICATION

(11) **EP 1 997 474 A1**
(43) Date of publication of application: **03.12.2008**
(21) Application number: 07714468.1
(22) Date of filing: 19.02.2007
(51) Int. Cl.: A61K 8/49, A61K 8/06, A61K 8/27, A61K 8/29, A61K 8/41, A61K 8/894, A61Q 17/04

(54) **WATER-IN-OIL EMULSION TYPE SUNSCREEN PREPARATION**

(30) Priority: 20.02.2006 JP 2006041832
(71) Applicant: Shiseido Co., Ltd., Tokyo 104-8010 (JP)
(72) Inventor: TAKAKURA, Yoshihito, Yokohama-shi, Kanagawa 2248558 (JP); YAMAGUCHI, Kazuhiro, Yokohama-shi, Kanagawa 2248558 (JP)
(74) Representative: Lippert, Stachow & Partner
(86) International application number: PCT/JP2007/052942
(87) International publication number: WO 2007/097275

(57) **Abstract**

An object of the present invention is to provide a water-in-oil type emulsion sunscreen cosmetic which can achieve excellent UV protection capability and have excellent effects in the prevention and inhibition stability of discoloration with the passage of time.

The water-in-oil type emulsion sunscreen cosmetic in the present invention is **characterized by** comprising 1 to 30 % by mass (a) hydrophobized titanium oxide and/or zinc oxide, 0.1 to 10 % by mass (b) phenylbenzimidazole sulfonate, 50 % by mass or more (c) triethanolamine with respect to the component (b), 0.1 to 10 % by mass (d) trimethylsiloxysilicate and further, if desired, 0.01 to 20 % by mass (e) silicone surfactant.

## Description

### RELATED APPLICATIONS

This application claims the priority of Japanese Patent Application No. 2006-041832 filed on February 20, 2006, which are incorporated herein by reference.

### FIELD OF THE INVENTION

The present invention relates to water-in-oil type emulsion sunscreen cosmetics. In particular, the present invention relates to water-in-oil type emulsion sunscreen cosmetics that have an excellent UV protection capability and have excellent effects in the prevention and inhibition stability of discoloration with the passage of time.

### BACKGROUND OF THE INVENTION

Generally, in order to block UV radiation to the skin and achieve high SPF (Sun Protection Factor) values, UV absorbers or UV scatterers (zinc oxide, titanium dioxide etc.) are blended in sunscreen cosmetics (refer to patent literatures 1 and 2, for example).

Phenylbenzimidazole sulfonic acid is an all-purpose UV absorber. However, there is a problem that discoloration with the passage of time occurs when phenylbenzimidazole sulfonic acid is used in combination with a hydrophobized UV scatterer such as zinc oxide and titanium dioxide in water-in-oil type emulsion sunscreen cosmetics.
Patent Literature 1 : Japanese Unexamined Patent Publication No. H10-120543.
Patent Literature 2 : PCT Japanese Translation Patent Publication No. 2002-521417.

### DISCLOSURE OF THE INVENTION

### PROBLEM TO BE SOLVED BY THE INVENTION

The object of the present invention is to provide water-in-oil type emulsion sunscreen cosmetics that solve the above-described problem, achieve an excellent UV shielding effect, and have excellent effects in the prevention and inhibition stability of discoloration with the passage of time.

### MEANS TO SOLVE THE PROBLEM

The present inventors have diligently studied to solve the above-described problem. As a result, the present inventors have found that an excellent UV shielding effect and excellent prevention and inhibition stability of discoloration with the passage of time could be stably achieved by neutralizing phenylbenzimidazole sulfonate with triethanolamine to dissolve in water phase and blending trimethylsiloxysilicate as a coating agent to oil phase (outer phase), thus leading to completion of the present invention.

The present invention provides water-in-oil type emulsion sunscreen cosmetics comprising: (a) 1 to 30 % by mass of hydrophobized titanium oxide and/or zinc oxide, (b) 0.1 to 10 % by mass of phenylbenzimidazole sulfonate, (c) 50 % by mass or more of triethanolamine with respect to said component (b), and (d) 0.1 to 10 % by mass of trimethylsiloxysilicate.

The present invention provides the above-described water-in-oil type emulsion sunscreen cosmetics further comprising : (e) 0.01 to 20 % by mass of silicone surfactant.

The present invention provides the above-mentioned water-in-oil type emulsion sunscreen cosmetics which do not substantially contain coloring pigment.

According to the present invention, water-in-oil type emulsion sunscreen cosmetics that can satisfactorily achieve an excellent UV protective effect which is a property of a UV absorber and have excellent effects in the prevention and inhibition stability of discoloration with the passage of time can be provided.

Hereinafter, the water-in-oil type emulsion sunscreen cosmetics of the present invention will be described in detail.

### BEST MODE FOR CARRYING OUT THE INVENTION

Hydrophobized titanium dioxide and/or zinc oxide, which is component (a) used in the present invention, is a UV scatterer, and it is efficiently dispersed into oil phase (outer phase) by hydrophobizing.

From the stand point of a UV-scattering effect, titanium dioxide and zinc oxide are preferably prepared into fine particles. The average primary particle size of titanium dioxide fine particles preferably includes 1 to 30 nm, more preferably 20 nm or less. The average primary particle size of zinc oxide fine particles preferably includes 1 to 40 nm, more preferably 30 nm or less. However, the sizes are not limited to these sizes.

The method for hydrophobizing treatment is not limited in particular, and a publicly known method can be used for treatment. Examples of hydrophobizing treatment include a treatment using silicones such as methylhydrogenpolysiloxane, methylhydrogenpolysiloxane/dimethylpolysiloxane coplymer, and dimethylpolysiloxane; a treatment using silane compound such as octyltriethoxysilane and hexyltrimethoxysilane; a treatment using fatty acid such as palmitic acid and stearic acid; a metallic soap treatment using such as alkali metal salt or alkaline earth metal salt of the above-mentioned fatty acid; and a fluorine treatment using such as diethanolamine salt of perfluoroalkylphosphate and perfluoroalkyltrimethoxysilane.

Hydrophobized titanium dioxide includes, for example, commercial products such as "TTO-S-4" and "TTO-V-4" (manufactured by ISHIHARA SANGYO KAISHA LTD.) and "MT-100TV" and "MT-014V" (manufactured by TAYCA CORPORATION.) Hydrophobized zinc oxide includes, for example, commercial products such as "FZO-50" (manufactured by ISHIHARA SANGYO KAISHA LTD.), "WSX-MZ-700" (manufactured by TAYCA CORPORATION), and "Z-Cote HP-1" (manufactured by BASF CORPARATION.) They can be preferably used in the present invention.

A blending amount of component (a) is 0.2 to 30 % by mass with respect to the cosmetics of the present invention, preferably 1 to 25 % by mass, and more preferably 10.1 to 25 % by mass. If the blending amount is less than 0.2 % by mass, a satisfactory UV protective effect cannot be achieved. On the other hand, if the blending amount exceeds 30 % by mass, there is a concern with discoloration and usability deterioration such as a smoothlessness with the passage of time. When the blending amount of component (a) is 10.1 % by mass or more, a highly excellent UV protective effect can be achieved, however at the same time, deterioration owing to combination use with component (b) described hereinafter is also significant. In the present invention, as described hereinafter, the above-mentioned discoloration can be stably prevented and inhibited from occurring by using component (b) as triethanolamine salt neutralized with component (c), and moreover, by using especially component (d) as a coating agent even if component (a) is blended in large amount. When hydrophobized zinc oxide is used as component (a), discoloration with the passage of time owing to combination use with component (b) is more significant compared with the case that hydrophobized titanium dioxide is used as component (a). In the present invention, even in such a case, discoloration can be stably and effectively prevented and inhibited from occurring by blending component (c) and moreover, by blending component (d).

Phenylbenzimidazole sulfonic acid, which is used as component (b), is a UV protection agent and includes, for example, commercial products such as "Neo Heliopan Hydro" (manufactured by Symrise AG) and "Eusolex232" (manufactured by Merck & Co.) They can be used desirably.

A blending amount of component (b) is 0.1 to 10 % by mass with respect to the cosmetic of the present invention, preferably 1 to 5 % by mass. If the blending amount is less than 0.1 % by mass, a satisfactory UV protection effect cannot be achieved. On the other hand, if the blending amount exceeds 10 % by mass, there is a concern that discoloration with the passage of time cannot not be completely inhibited from occurring.

Triethanolamine, which is used as component (c), is a neutralizer for component (b). Discoloration of system tends to occur when component (a) and component (b) are used together, however, such discoloration can be effectively prevented and inhibited from occurring by using component (b) as triethanolamine salt neutralized with component (c). In case zinc oxide is particularly used as component (a), a degree of discoloration tends to increase more than the case of using titanium dioxide. In the present invention, however, even in such a case, such discoloration can be effectively prevented and inhibited from occurring by blending component (c) to use component (b) as triethanolamine hydrochloride.

A blending amount of component (c) is 50 % by mass or more with respect to component (b), preferably 55 % by mass or more, more preferably 60 % by mass or more. If the blending amount of component (c) with respect to component (b) is less than 50 % by mass, component (c) cannot fully neutralize component (b) and there is a concern of crystal precipitation. The upper limit of the blending amount of component (c) is not particularly limited, however, it is generally preferred that the blending amount of component (c) which is used only for neutralizing component (b) is about 65 % by mass or less with respect to component (b) because excess amount of component (c) may rise pH of system.

Trimethylsiloxysilicate, which is used as component (d), is oil-soluble polymer as a coating agent and includes commercial products such as "X-21-5249" (manufactured by Shin-Etsu Chemical Co. Ltd.), which can be used desirably.

A blending amount of component (d) is 0.1 to 10 % by mass with respect to the cosmetic of the present invention, preferably 0.5 to 5 % by mass. If the blending amount is less than 0.1 % by mass, a satisfactory prevention and inhibition effect of discoloration cannot be achieved. On the other hand, if the blending amount exceeds 10 % by mass, there is a concern with usability deterioration such as a smoothlessness and a coated feeling.

In the present invention, it is also desirable that a (e) silicone surfactant is blended as an emulsifying agent. The silicone surfactant is not limited in particular so far as it is usable in water-in-oil emulsion systems. Examples include poly(oxyethylene/oxypropylene) methylpolysiloxane copolymer, polyoxyethylene methylpolysiloxane copolymer, silicone-chain branched-type methylpolysiloxane copolymer, alkyl-chain branched-type polyoxyethylene methylpolysiloxane copolymer, alkyl-chain/silicone-chain branched-type polyoxyethylene methylpolysiloxane copolymer, crosslinked-type polyoxyethylene methylpolysiloxane, alkyl-containing crosslinked-type polyoxyethylene methylpolysiloxane, branched-type polyglycerin-modified silicone, crosslinked-type polyglycerin-modified silicone, alkyl-containing crosslinked-type polyglycerin-modified silicone, and alkyl group branched-type polyglycerin-modified silicone. However, the silicone surfactant is not limited to these.

Examples of the above-described poly(oxyethylene/oxypropylene)methylpolysiloxane copolymers include PEG/PPG-20/22 butyl ether dimethicone ("KF-6012", manufactured by Shin-Etsu Chemical Co., Ltd.), PEG/PPG-20/20 dimethicone ("BY22-008M", manufactured by Toray Dow Coming Silicone Co., Ltd.), lauryl PEG/PPG-18 methicone ("5200 Formulation Aid", manufactured by Dow Coming Toray Co., Ltd.), PEG/PPG-19/19 dimethicone("5330 Fluid", manufactured by Dow Coming Toray Co., Ltd.), and PEG/PPG-15/15 dimethicone ("5330 Fluid", manufactured by Dow Coming Toray Co., Ltd.).

Examples of polyoxyethylene methylpolysiloxane copolymers include PEG-11 methyl ether dimethicone ("KF-6011", manufactured by Shin-Etsu Chemical Co., Ltd.), PEG-9 dimethicone ("KF-6013", manufactured by Shin-Etsu Chemical Co., Ltd.), PEG-3 dimethicone ("KF-6015", manufactured by Shin-Etsu Chemical Co., Ltd.), PEG-9 methyl ether dimethicone ("KF-6016", manufactured by Shin-Etsu Chemical Co., Ltd.), PEG-10 dimethicone ("KF-6017", manufactured by Shin-Etsu Chemical Co., Ltd.), PEG-11 methyl ether dimethicone ("KF-6018", manufactured by Shin-Etsu Chemical Co., Ltd.), PEG-9 dimethicone ("KF-6019", manufactured by Shin-Etsu Chemical Co., Ltd.), and PEG-12 dimethicone ("SH3771 M ", "SH3772 M ", "SH3773 M ", "SH3775 M ", etc., manufactured by Dow Coming Toray Co., Ltd.).

Examples of silicone-chain branched-type methylpolysiloxane copolymers include PEG-9 polydimethylsiloxyethyl dimethicone ("KF-6028", manufactured by Shin-Etsu Chemical Co., Ltd.).

Examples of alkyl-chain branched-type polyoxyethylene methylpolysiloxane copolymers include PEG/PPG-10/3 oleyl ether dimethicone ("KF-6026", manufactured by Shin-Etsu Chemical Co., Ltd.).

Examples of alkyl-chain/silicone-chain branched-type polyoxyethylene methylpolysiloxane copolymers include lauryl PEG-9 polydimethylsiloxyethyl dimethicone ("KF-6038", manufactured by Shin-Etsu Chemical Co., Ltd.).

Examples of crosslinked-type polyoxyethylene methylpolysiloxanes include dimethicone (dimethicone/(PEG-10/15)) crosspolymer ("KSG-210", manufactured by Shin-Etsu Chemical Co., Ltd.) and cyclomethicone/PEG-12 dimethicone dimethicone crosspolymer ("9011 Silicone Elastomer Blend", manufactured by Toray Dow Coming Silicone Co., Ltd.).

Examples of alkyl-containing crosslinked-type polyoxyethylene methylpolysiloxanes include mineral oil/PEG-15 lauryl dimethicone crosspolymer ("KSG-310", manufactured by Shin-Etsu Chemical Co., Ltd.), isododecane/PEG-15 lauryl dimethicone crosspolymer ("KSG-320", manufactured by Shin-Etsu Chemical Co., Ltd.), trioctanoin/PEG-15 lauryl dimethicone crosspolymer ("KSG-330", manufactured by Shin-Etsu Chemical Co., Ltd.), and squalane/PEG-15 lauryl dimethicone crosspolymer/PEG-10 lauryl dimethicone crosspolymer ("KSG-340", manufactured by Shin-Etsu Chemical Co., Ltd.).

Examples of branched-type polyglycerin-modified silicones include polyglyceryl-3 disiloxane dimethicone ("KF-6100", manufactured by Shin-Etsu Chemical Co., Ltd.) and polyglyceryl-3 polydimethylsiloxyethyl dimethicone ("KF-6104", manufactured by Shin-Etsu Chemical Co., Ltd.).

Examples of crosslinked-type polyglycerin-modified silicones include dimethicone/(dimethicone/polyglycerin-3) crosspolymer ("KSG-710", manufactured by Shin-Etsu Chemical Co., Ltd.).

Examples of alkyl-containing crosslinked-type polyglycerin-modified silicones include mineral oil/(lauryl dimethicone/polyglycerin-3) crosspolymer ("KSG-810", manufactured by Shin-Etsu Chemical Co., Ltd.), isododecane/(lauryl dimethicone/polyglycerin-3) crosspolymer ("KSG-820, manufactured by Shin-Etsu Chemical Co., Ltd."), trioctanoin/(lauryl dimethicone/polyglycerin-3) crosspolymer ("KSG-830", manufactured by Shin-Etsu Chemical Co., Ltd.), and squalane/(lauryl dimethicone/polyglycerin-3) crosspolymer ("KSG-840", manufactured by Shin-Etsu Chemical Co., Ltd.).

Examples of alkyl group branched-type polyglycerin-modified silicones include lauryl polyglyceryl-3 polydimethylsiloxyethyl dimethicone ("KF-6105", manufactured by Shin-Etsu Chemical Co., Ltd.).

Among them, polyoxyethylene methylpolysiloxane copolymer, poly(oxyethylene/oxypropylene) methylpolysiloxane copolymer, silicone-chain branched-type methylpolysiloxane copolymer, and alkyl-chain/silicone-chain branched-type polyoxyethylene methylpolysiloxane copolymer are desirably used.

The blending amount of component (e) in the sunscreen cosmetics of the present invention is preferably 0.01 % by mass at the lower limit, more preferably 0.1 % by mass or more, and further more preferably 0.5 % by mass or more. The upper limit is 20 % by mass or less and more preferably 10 % by mass or less. If the blending amount is less than 0.01 % by mass, the stability of cosmetics tends to become poorer. On the other hand, if the blending amount largely exceeds 20 % by mass, a sticky feeling is generated and the feeling in use tends to become poorer.

In the present invention, from the viewpoint of dyeing property, it is preferred that coloring pigments such as iron oxide red, iron oxide yellow, iron oxide black, chromium oxide, ultramarine, Prussian blue, and carbon black, are not substantially contained.

In the water-in-oil type emulsion sunscreen cosmetics of the present invention, it is preferred that the oil phase (outer phase) is 20 to 80 % by mass and the water phase (inner phase) is 80 to 20 % by mass.

In the cosmetics of the present invention, in addition to the above-described components, other components normally used in cosmetics can be blended as necessary so far as the objectives and effects of the present invention are not undermined. Examples of these components include water-soluble polymers, oil-soluble polymers (other than the above-described component (d)), polymer powders, emulsifying agents (other than the above-described component (e)), waxes, alcohols, liquid fats, ester oils, hydrocarbon oils, silicon oils, fatty acids, higher alcohols, fatty acid esters, drugs, UV absorbers (other than the above-described component (b)), UV scatterers (other than the above-described component (a)), and organic-modified clay minerals. However, the components are not limited by these

### examples.

Examples of water-soluble polymers include a homopolymer and copolymers of 2-acrylamido-2-methyl propane sulfonic acid (hereinafter abbreviated as "AMPS"). The copolymer comprises comonomers such as vinylpyrrolidone, acrylamide, sodium acrylate, and hydroxyethyl acrylate. Examples include AMPS homopolymer, vinylpyrrolidone/AMPS copolymer, dimethylacrylamide/AMPS copolymer, acrylamide/AMPS copolymer, and sodium acrylate/AMPS copolymer.

Further examples include carboxyvinyl polymer, ammonium polyacrylate, sodium polyacrylate, sodium acrylate/alkyl acrylate/sodium methacrylate/alkyl methacrylate copolymer, carrageenan, pectin, mannan, curdlan, chondroitin sulfate, starch, glycogen, gum arabic, sodium hyaluronate, tragacanth gum, xanthan gum, mucoitin sulfate, hydroxyethyl guar gum, carboxymethyl guar gum, guar gum, dextran, kerato sulfate, locust bean gum, succinoglucan, chitin, chitosan, carboxymethyl chitin, and agar.

Examples of oil-soluble polymers (other than the above-described component (d)) include alkyl-modified silicone and polyamide-modified silicone.

Examples of polymer powders include dimethicone crosspolymer, (dimethicone/vinyldimethicone) crosspolymer, polymethylsilsesquioxane, polyethylene, and polymethylmethacrylate.

Examples of waxes include beeswax, candelilla wax, carnauba wax, lanolin, lanolin oil, and jojoba wax.

Examples of emulsifying agents (other than the above-described component (e)) include glycerin fatty acid ester, polyglycerin fatty acid ester, polyoxyethylene glycerin fatty acid ester, sorbitan fatty acid ester, and polyoxyethylene sorbitan fatty acid ester.

Examples of alcohols include lower alcohols such as ethanol and isopropanol; higher alcohols such as isostearyl alcohol, octyldodecanol, and hexyldecanol; and polyhydric alcohols such as ethylene glycol, propylene glycol, 1,3-butylene glycol, dipropylene glycol, and polybutylene glycol.

Examples of liquid fat include avocado oil, camellia oil, turtle oil, macadamia nut oil, corn oil, mink oil, olive oil, rapeseed oil, egg oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, paulownia oil, Japanese tung oil, jojoba oil, germ oil, and triglycerin.

Examples of ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, acetylated lanolin, isocetyl stearate, isocetyl isostearate, isononyl isononate, cholesteryl 12-hydroxystearate, ethylene glycol di-2-ethylhexanoate, dipentaerythritol fatty acid ester, N-alkylglycol monoisostearate, neopentyl glycol dicaprylate, diisostearyl malate, glyceryl di-2-heptylundecanoate, trimethylolpropane tri-2-ethylhexanoate, trimethylolpropane triisostearate, pentaerythritol tetra-2-ethylhexanoate, glyceryl tri-2-ethylhexanoate, glyceryl trioctanoate, glyceryl triisopalmitate, trimethylolpropane triisostearate, cetyl 2-ethylhexanoate, 2-ethylhexyl palmitate, glyceryl trimyristate, glyceride tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate.

Examples of hydrocarbon oils include liquid paraffin, ozokerite, squalane, pristane, paraffin, ceresin, squalene, petrolatum, microcrystalline wax, polyethylene wax, and Fischer-Tropsch wax.

Examples of silicon oils include dimethylpolysiloxane, octamethylsiloxane, decamethyltetrasiloxane, methylhydrogenpolysiloxane, methylphenylpolysiloxane, hexamethylcyclotrisiloxane, octamethylcyclotetrasiloxane, and decamethylcyclopentasiloxane.

Examples of fatty acids include lauric acid, myristic acid, palmitic acid, stearic acid, behenic acid, and arachidonic acid.

Examples of higher alcohols include lauryl alcohol, myristyl alcohol, cetyl alcohol, stearyl alcohol, behenyl alcohol, arachyl alcohol, batyl alcohol, chimyl alcohol, carnaubyl alcohol, ceryl alcohol, koryanyl alcohol, myricyl alcohol, lacceryl alcohol, elaidyl alcohol, isostearyl glyceryl ether, octyl alcohol, triancotyl alcohol, cerakyl alcohol, cetostearyl alcohol, oleyl alcohol, lanolin alcohol, hydrogenated lanolin alcohol, hexyldecanol, and octyldecanol.

Examples of fatty acid esters include myristyl myristate, cetyl palmitate, cholesteryl stearate, and 2-octyldodecyl beeswax fatty acid.

Examples of drugs include L-ascorbic acid and its salt derivatives, glycyrrhizic acid and its derivatives such as dipotassium glycyrrhizate and monoammonium glycyrrhizate, glycyrrhetinic acid and its derivatives such as stearyl glycyrrhetinate, allantoin, tranexamic acid and its salt derivatives, alkoxysalicylic acid and its salt derivatives, glutathione and its salt derivatives, allantoin, and azulene.

Examples of UV absorbers (other than the above-described component (b)) include cinnamic acid derivatives such as ethylhexyl methoxycinnamate, isopropyl methoxycinnamate, and isoamyl methoxycinnamate; PABA derivatives such as para-aminobenzoic acid (hereinafter abbreviated as "PABA"), ethyl PABA, ethyl dihydroxypropyl PABA, ethylhexyl dimethyl PABA, and glyceryl PABA; salicylic acid derivatives such as homosalate, ethylhexyl salicylate, dipropylene glycol salicylate, and TEA salicylate; benzophenone derivatives such as benzophenone-1, benzophenone-2, benzophenone-3 or oxybenzone, benzophenone-4, benzophenone-5, benzophenone-6, benzophenone-8, benzophenone-9, and benzophenone-12; benzylidene camphor derivatives such as 3-benzylidene camphor, 4-methylbenzylidene camphor, benzylidene camphor sulfonic acid, camphor benzalkonium methosulfate, terephthalylidene dicamphor sulfonic acid, and polyacrylamidomethyl benzylidene camphor; triazine derivatives such as anisotriazine, ethylhexyl triazone, diethylhexyl butamido triazone, and 2,4,6-tris(diisobutyl 4'-aminobenzalmalonate)-s-triazine; phenylbenzimidazole derivatives such as disodium phenyldibenzimidazole tetrasulfonate; phenylbenzotriazol derivatives such as drometrizole trisiloxane and methylene bis-benzotriazolyl tetramethylbutylphenol; anthranilic derivatives such as menthyl anthranilate; imidazoline derivatives such as ethylhexyl dimethoxybenzylidene dioxoimidazoline propionate; benzalmalonate derivatives such as polyorganosiloxanes with benzalmalonate functional groups; 2-cyano-3,3-diphenyl-2-propenoic acid-2-ethylhexyl ester; 4,4-diarylbutadiene derivatives such as 1,1-dicarboxy(2,2'-dimethylpropyl)-4,4-diphenylbutadiene and octocrylene.

Examples of UV scatterers (other than the above-described component (a)) include hydrophobized inorganic pigments such as kaolin and calcium carbonate.

Examples of organic-modified clay minerals include quaternary ammonium cation modified clay minerals.

The water-in-oil type emulsion sunscreen cosmetics of the present invention include products such as emulsion type and cream type. These products can be prepared by the conventional method by mixing the above-described essential components and the components that are normally blended in cosmetics.

Hereinafter, the present invention will be described in further detail with reference to examples. However, the present invention is not limited by the following examples. All the blending amounts are shown in % by mass.

### EXAMPLES

### Examples 1 to 3, Comparative examples 1 to 5

Sunscreen cosmetics were prepared according to the formulations in the following Table 1.

To be more precise, the sunscreen cosmetics were prepared by adding (8), as part B, to (1) to (7), as part A, to be dispersed homogeneously, and consequently adding thereto (9) to (11), as part C, to be dispersed homogeneously, and then gradually adding thereto (12) to (19), as part D, to be emulsified.

### Prevention and inhibition stability of discoloration

The prevention and inhibition stabilities of discoloration in the respective obtained sunscreen cosmetics (samples) were evaluated according to the following evaluation criteria. The results are shown in Table 1.

### Test method

The color hues of the respective examples, which were fulfilled in transparent grass tubes and left at rest at 0 °C and at 50 °C for one month, were compared and evaluated according to the following evaluation criteria.

### Evaluation criteria

○: There was no difference in the color hues between the example preserved at 0 °C and that at 50 °C.
Δ: While redness could be observed in the clear layer of the example preserved at 50 °C compared with that at 0 °C in creamed state, the difference in the color hues disappeared by shaking them to be homogenous.
X: Clear differences in the color hues could be observed between the example preserved at 0 °C and that at 50 °C even by shaking them to be homogenous.

**Table 1**

| | Examples | | | Comparative examples | | | | |
|---|---|---|---|---|---|---|---|---|
| | 1 | 2 | 3 | 1 | 2 | 3 | 4 | 5 |
| (1) Decamethylcyclopentasiloxane | Balance | Balance | Balance | Balance | Balance | Balance | Balance | Balance |
| (2) Dimethylpolysiloxane (*1) | 2 | 2 | 2 | 2 | 2 | 2 | 2 | 2 |
| (3) Isononyl isononate | 3 | 3 | 3 | 3 | 3 | 3 | 3 | 3 |
| (4) Trimethylsiloxysilicate [component(d)] | 2.5 | 2.5 | 5 | 2.5 | 2.5 | - | 2.5 | - |
| (5) Ethylhexyl methoxycinnamate (*2) | 5 | 5 | 5 | 5 | 5 | 5 | 5 | 5 |
| (6) Bis(resorcinyl)triazine (*3) | 0.5 | 0.5 | 0.5 | 0.5 | 0.5 | 0.5 | 0.5 | 0.5 |
| (7) Alkyl-chain/silicone-chain branched-type polyoxyethylene methylpolysiloxane copolymer (*4) [component(e)] | 1 | 1 | 1 | 1 | 1 | 1 | 1 | 1 |
| (8) Organic-modified clay minerals (*5) | 0.2 | 0.2 | 0.2 | 0.2 | 0.2 | 0.2 | 0.2 | 0.2 |
| (9) Dextrin plamitate-treated zinc oxide (*6) [component(a)] | 15 | - | 12 | 31 | 15 | 15 | 15 | 15 |
| (10) Stearic acid-treated titanium dioxide(*7) [component(a)] | - | 15 | 5 | - | - | - | - | - |
| (11) Polymethylsilsesquioxane | 6 | 6 | - | - | 6 | 6 | 6 | 6 |
| (12) Ion-exchanged water | 24.3 | 24.3 | 28.3 | 14.3 | 11.5 | 24.3 | 25.65 | 25.65 |
| (13) Phenylbenzimidazole sulfonic acid (*8) [component(b)] | 3 | 3 | 3 | 3 | 11 | 3 | 3 | 3 |
| (14) Triethanolamine [component(c)] | 1.8 | 1.8 | 1.8 | 1.8 | 6.6 | 1.8 | | - |
| (15) Sodium hydroxide | - | - | - | - | - | - | 0.45 | 0.45 |
| (16) Glycerin | 1 | 1 | 1 | 1 | 1 | 1 | 1 | 1 |
| (17) Trisodium edtate | 0.1 | 0.1 | 0.1 | 0.1 | 0.1 | 0.1 | 0.1 | 0.1 |
| (18) Ethyl alcohol (95%) | 5 | 5 | 5 | 5 | 5 | 5 | 5 | 5 |
| (19) Phenoxyethanol | 0.35 | 0.35 | 0.35 | 0.35 | 0.35 | 0.35 | 0.35 | 0.35 |
| Prevention and suppression stability of discoloration | ○ | ○ | ○ | Δ | Δ | Δ | Δ | X |

The products mentioned below were used as the following compounds in Table 1.

Dimethylpolysiloxane (*1) : "KF96A-6T" (manufactured by Shin-Etsu Chemical Co.,Ltd.), ethylhexyl methoxycinnamate (*2) : "PARSOL MCX" (manufactured by DSM Nutrition Japan K.K.), bis ( resorcinyl ) triazine (*3): "TINOSORB S" (manufactured by Ciba Specialty Chemicals Inc.), alkyl-chain/silicone-chain branched-type polyoxyethylene methylpolysiloxane copolymer (*4) : "KF6038" (manufactured by Shin-Etsu Chemical Co.,Ltd.), organic-modified clay minerals (*5) : "Bentone 38V CG" (manufactured by Elementis Specialties, Inc.), dextrin palmitate-treated zinc oxide (*6) : "WSX-MZ-700" (manufactured by TAYCA CORPORATION), stearic acid-treated titanium dioxide (*7) : "TTO-V-4" (manufactured by ISHIHARA SANGYO KAISHA LTD.), phenylbenzimidazole sulfonic acid (*8) : "Eusolex232" (manufactured by Merck & Co., Inc.).

As is clear from the result in Table 1, the water-in-oil type emulsion sunscreen cosmetics of the present invention was confirmed to be excellent in prevention and inhibition stability of discoloration.

## Claims

1. A water-in-oil type emulsion sunscreen cosmetic comprising:
(a) 1 to 30 % by mass of hydrophobized titanium oxide and/or zinc oxide;
(b) 0.1 to 10 % by mass of phenylbenzimidazo le sulfonate;
(c) 50 % by mass or more of triethanolamine with respect to said component (b); and
(d) 0.1 to 10 % by mass of trimethylsiloxysilicate.

2. The water-in-oil type emulsion sunscreen cosmetic according to claim 1 further comprising:
(e) 0.01 to 20 % by mass of silicone surfactant.

3. The water-in-oil type emulsion sunscreen cosmetics according to claims 1 or 2 wherein coloring pigments are not substantially contained.
